# EUROPEAN PATENT SPECIFICATION

(11) **EP 2 396 336 B1**
(45) Date of publication and mention of the grant of the patent: **15.05.2013**
(21) Application number: 10703948.9
(22) Date of filing: 10.02.2010
(51) Int. Cl.: C07D 493/10

(54) **PROCESS FOR THE PREPARATION OF 5-/6-NITROFLUORESCEIN**
VERFAHREN ZUR HERSTELLUNG VON 5-/6-NITROFLUORESCEIN
PROCEDE DE PREPARATION DE 5-/6-NITROFLUORESCEINE

(30) Priority: 12.02.2009 GB 0902286
(43) Date of publication of application: 21.12.2011
(73) Proprietor: Norgine BV, 1101 CA Amsterdam Zuid-Oost (NL)
(72) Inventor: WHARTON, Stuart, Cheshire CH2 3PR (GB); ROBERTS, Tomos, Huw, Gwynedd LL57 2LA (GB)
(74) Representative: Maughan, Sophie Louise
(86) International application number: PCT/GB2010/050206
(87) International publication number: WO 2010/092382

(56) References cited:
- WO-A1-97/39064
- DATABASE WPI Week 198048 Thomson Scientific, London, GB; AN 1980-85839C XP002576161 -& SU 727 663 A1 (KIROV POLY) 15 April 1980 (1980-04-15)
- COONS A H ET AL: "Localization of antigen in tissue cells. II. Improvements in a method for the detection of antigen by means of fluorescent antibody" JOURNAL OF EXPERIMENTAL MEDICINE 1950, vol. 91, no. 1, 1950, pages 1-13, XP002576162 ISSN: 0022-1007 cited in the application
- CIHELNIK S ET AL: "Solvent-free synthesis of sulfonephthaleins, sulfonefluoresceins and fluoresceins under microwave irradiation" COLLECTION OF CZECHOSLOVAK CHEMICAL COMMUNICATIONS, vol. 67, 1 January 2002 (2002-01-01), pages 1779-1789, XP009034707 ISSN: 0010-0765 DOI: DOI:10.1135/CCCC20021779

## Description

The present invention relates to an improved process for the preparation of 5-/6 nitrofluorescein.

5-Nitrofluorescein (3',6'-dihydroxy-6-nitrospiro[2-benzofuran-3,9'-xanthene]-1-one) is a key intermediate in the synthesis of various fluorescent compounds such as 5-aminofluorescein (5-amino-3',6'-dihydroxy-3H-spiro[isobenzofuran-1,9'-xanthen]-3-one), fluorescein 5-isothiocyanate (FITC; 3',6'-dihydroxy-5-isothiocyanato-3*H-*spiro[isobenzofuran-1,9'-xanthen]-3-one), and fluorescein lisicol (previously known as cholyl-lysylfluorescein or CLF; N6-({3',6'-dihydroxy-3-oxospiro[isobenzofuran-1(3H),9'-xanthen]-5-yl}thiocarbamoyl)-N2-(3,7,12-trihydroxy-5-cholan-24-oyl)-L-lysine). Fluorescein 5-isothiocyanate has a wide range of biological applications including use as a fluorescent labelling agent for proteins, as a fluorescent reagent for protein tracing, and as a reagent in a fluorescent antibody technique for the rapid identification of pathogens. In addition, the use of fluorescent bile acid derivatives, and CLF in particular, in a method for the determination of the liver function of a human or animal subject is described in EP1,003,458 (Norgine Europe BV).

A.H. Coons and M.H. Kaplan (Journal of Experimental Medicine 1950, 91, 1 - 13) describe the synthesis of 5-/6-nitrofluorescein by the thermal condensation of a dry mixture of 4-nitrophthalic acid and resorcinol at 195 - 200°C for 12 to 18 hours to give a 98% yield of crude 5-/6-nitrofluorescein. No indication is given in the paper regarding the isomer ratio or the degree of conversion. However, in the subsequent acetylation and separation of isomers, Coons and Kaplan report a 26% yield of 5-nitrofluorescein diacetate.

Importantly, the reaction mixture becomes a solid mass during the thermal condensation reaction and recovery of the product proves to be extremely difficult when the reaction is scaled up. Coons and Kaplan report the reaction on a 100 gram scale, i.e. 100g 4-nitrophthalic acid and 100g resorcinol as starting materials, and describe the solidified melt being chipped from the beaker in which the reaction was conducted. However, when the scale of the reaction is increased, and when the reaction is conducted under an inert atmosphere, it has been our experience that recovery inevitably requires destruction of the reaction vessel in order that the solid product can be chipped out prior to further processing. This becomes uneconomic when the reaction is scaled up to produce kilo quantities of the intermediate.

Furthermore, and importantly, we have found that there is a serious health and safety concern on scale up due to the risk of explosion. In our investigations, an uncontrollable and violent exothermic reaction can occur when the reaction is carried out on above a 50g - 100g scale.

In summary, the prior art process is unworkable on scale up and there is a long-felt need in the industry to provide a process which can be used safely to produce kilo or multi-kilo batches of 5-/6-nitrofluorescein and which does not require protracted reaction times or elaborate work up procedures.

A main object of the present invention is to provide a high yielding process for the preparation of 3',6'-dihydroxy-6-nitrospiro[2-benzofuran-3,9'-xanthene]-1-one and 3',6'-dihydroxy-5-nitrospiro[2-benzofuran-3,9'-xanthene]-1-one.

Another object of the present invention is to provide a process for the preparation of 3',6'-dihydroxy-6-nitrospiro[2-benzofuran-3,9'-xanthene]-1-one and 3',6'-dihydroxy-5-nitrospiro[2-benzofuran-3,9'-xanthene]-1-one with relatively short reaction times.

Yet another object of the present invention is to provide such a process to prepare a mixture of 3',6'-dihydroxy-6-nitrospiro[2-benzofuran-3,9'-xanthene]-1-one and 3',6'-dihydroxy-5-nitrospiro[2-benzofuran-3,9'-xanthene]-1-one in substantially pure form without elaborate work up procedures. The term "substantially pure" refers to material that is not less than 85% pure. A more preferred purity for the product derived from the present process is not less than 89% pure and typically the product so produced has a purity of about 89 to 94%. Purity can be determined by a number of conventional analytical methods, including HPLC and n.m.r. analyses.

The present invention may be described by the following reaction scheme:- 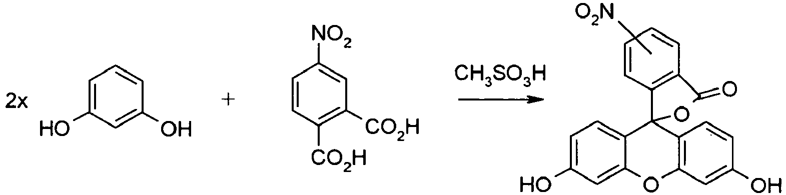 Accordingly, there is provided a process for the preparation of a mixture of 3',6'-dihydroxy-6-nitrospiro[2-benzofuran-3,9'-xanthene]-1-one and 3',6'-dihydroxy-5-nitrospiro[2-benzofuran-3,9'-xanthene]-1-one comprising reacting 4-nitrophthalic acid or 4-nitrophthalic anhydride with benzene-1,3-diol in methanesulphonic acid comprising the steps of:-
(a) reacting 4-nitrophthalic acid or 4-nitrophthalic anhydride with benzene-1,3-diol in methanesulphonic acid;
(b) quenching the reaction in step (a) with a solvent to precipitate product;
(c) isolating the precipitate;
(d) heating the precipitate in water in order to hydrolyse any methansulphonic acid ester present.

It should be noted that 4-nitrophthalic anhydride can be used interchangeably with 4-nitrophthalic acid as a starting material in this condensation reaction. Indeed, mixtures of 4-nitrophthalic acid and 4-nitrophthalic anhydride could be used as starting materials if required.

SU727663 discloses a process for the preparation of a mixture of 5 and 6-isomers of nitrofluorescein comprising reacting 4-nitrophthalic acid with resorcinol in orthophosphoric acid, quenching the reaction in 0.06 HCL, isolating the precipitate and drying, but does not disclose the use of methanesulfonic acid.

Carrying out the condensation reaction in methanesulphonic acid instead of by thermal fusion provides much milder and safer reaction conditions over a very much shorter reaction time.

Any potential exotherm during the condensation reaction can be controlled by controlling the reaction temperature, optionally by heating with a water bath. Preferably the reaction is carried out at a temperature in the range of from 60° to 120°C, more preferably above 90°C. A particularly preferred temperature range for carrying out the condensation reaction is 95° to 100°C.

Preferably the methanesulphonic acid is substantially pure. More preferably the methanesulphonic acid is not less than 95% w/w. In a particularly preferred embodiment the methanesulphonic acid is not less than 98% w/w.

Preferably, the reaction environment is selected such that the product is precipitated from the reaction mixture. Accordingly, the reaction mixture is more preferably quenched with a solvent and more preferably quenched to precipitate the product from the reaction mixture. In a particularly preferred embodiment the solvent used comprises an aqueous medium. In a further preferred embodiment the reaction mixture is added to water to precipitate the product. The final step of the process is one that will enable the product to be isolated from the reaction mixture and is preferably filtration. However, the product may also be obtained by centrifugation of the reaction mixture after precipitation of the product.

When water is used as a solvent to quench the reaction, the temperature of the water prior to quenching the reaction mixture is preferably 0° to 10°C, and preferably the temperature of the aqueous phase is maintained at 30°C or less during the addition process.

Preferably the ratio of water to methanesulphonic acid in the quench is 4.5 w/w or less, and more preferably 3 w/w or less.

In a particularly preferred embodiment any methanesulphonic acid ester present in the product is hydrolysed. Therefore, especially preferred is when the precipitated product is preferably heated in water at 80° to 100°C, more preferably with agitation. The aqueous hydrolysis step is preferably repeated until the amount of residual methanesulphonic acid ester is below a pre-determined level. A suitable pre-determined level for the amount of residual methanesulphonic acid ester is 5% or less. The product from this hydrolysis step may be isolated by filtration or centrifugation.

Preferably the isolated product is dried in vacuum at up to about 65° to 70°C.

In the condensation reaction, the volume of methanesulphonic acid in the reactants, based on the weight of 4-nitrophthalic acid or 4-nitrophthalic anhydride, is preferably 2 to 10 v/w and more preferably 3 to 5 v/w.

In order to achieve the formation of the desired mixed nitrofluorescein isomers, a reactor containing 4-nitrophthalic acid and resorcinol (benzene-1,3-diol) in methanesulphonic acid is heated, preferably to over 90°C and more preferably 95 - 100°C. Failure to maintain the temperature or reaction time may result in incomplete reaction.

During the synthesis the end point of the reaction (determined as the time when 4-nitrophthalic acid HPLC area is preferably less than 2% area) is determined by in-process analysis of the reaction mixture. This limit is important in obtaining intermediate product that meets specification.

Suitable HPLC conditions include using a Waters (RTM) XBridgeShield RP18 column 100 mm in length, 4.6 mm in diameter with a particle size of 3.5 µm. The mobile phase may be a water containing 0.1% H₃PO₄ and/or acetonitrile containing 0.1% H₃PO₄ with a mixing ratio of between 100% aqueous phase and 100% acetonitrile phase over 25 minutes.

Once the reaction is complete it is allowed to cool and may be quenched by pouring into a solvent, for example water. The resulting solid is filtered and transferred back to the reaction vessel and water is added and the mixture hydrolyzed, preferably at 95 - 100 °C, and preferably for at least 30 minutes. On cooling the solid is isolated, preferably by filtration or centrifugation, recharged to the reaction vessel and hydrolysed with a further portion of water at, again preferably at 95 - 100 °C for 30 minutes. Failure to maintain the optimum temperature and time for the hydrolysis may result in the incomplete hydrolysis of any methanesulphonic acid ester present. The reaction is again allowed to cool and the solid isolated by filtration or centrifugation and dried to constant weight in a vacuum oven at 65-70 °C. Failure to maintain the temperature may result in decomposition of the product in the event of overheating.

In summary, what appeared to be methanesulphonic acid contamination of the crude condensation product was found to be due to the formation of methanesulphonic acid ester of one or both phenol groups. Although this esterfied product is substantially insoluble in water it has unexpectedly and counter-intuitively been found that complete hydrolysis of any methanesulphonic acid ester can be achieved simply by heating or refluxing the precipitate from the cold water quench in water and repeating this aqueous hydrolysis if necessary. Aqueous hydrolysis avoids digestion with hydrochloric acid, which is a requirement after the thermal condensation process A.H. Coons and M.H. Kaplan (Journal of Experimental Medicine 1950, 91, 1 - 13).

The isomer ratio of 5-/6-nitrofluorescein produced in the methanesulphonic acid catalysed condensation is in the order of about 60:40 to about 70:30 5-nitrofluorescein : 6-nitrofluorescein.

A mixture of 5-/6-nitrofluorescein prepared according to the present invention has a number of applications. For example, as referred to above, it can be used to synthesise fluorescein 5-isothiocyanate according to reaction Scheme 1 below:- 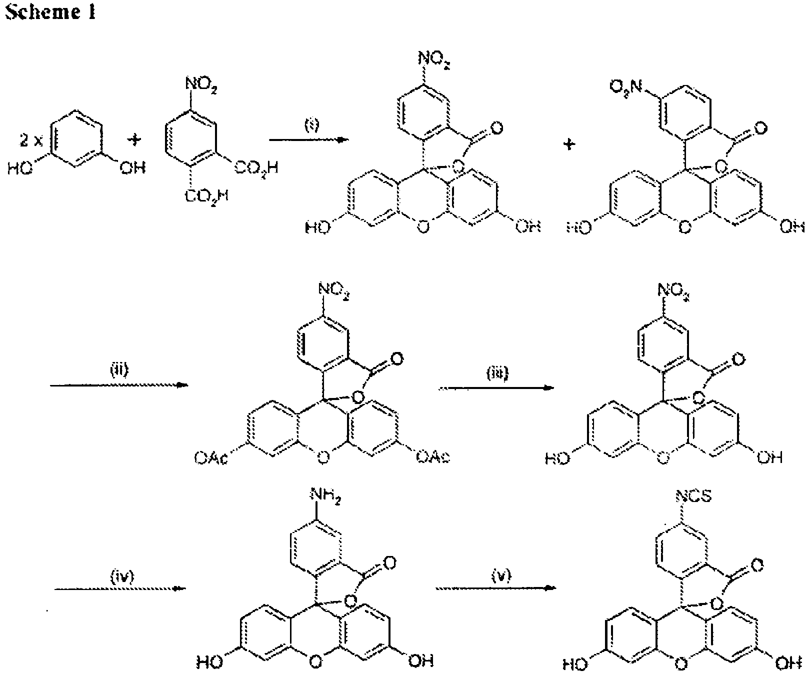 Reagents: (i) Methanesulphonic acid, (ii) AcO₂, reflux. Then AcOH recryst, (iii) MeOH, NaOH, (iv) Pd/C, cyclohexene, methanol, reflux, (v) thiophosgene, acetone.

Fluorescein 5-isothiocyanage (FITC), and thus 5-/6 nitrofluorescein, can be used to synthesise fluorescein lisicol (previously known as cholyl-lysylfluorescein (CLF))according to Scheme 2 shown below.

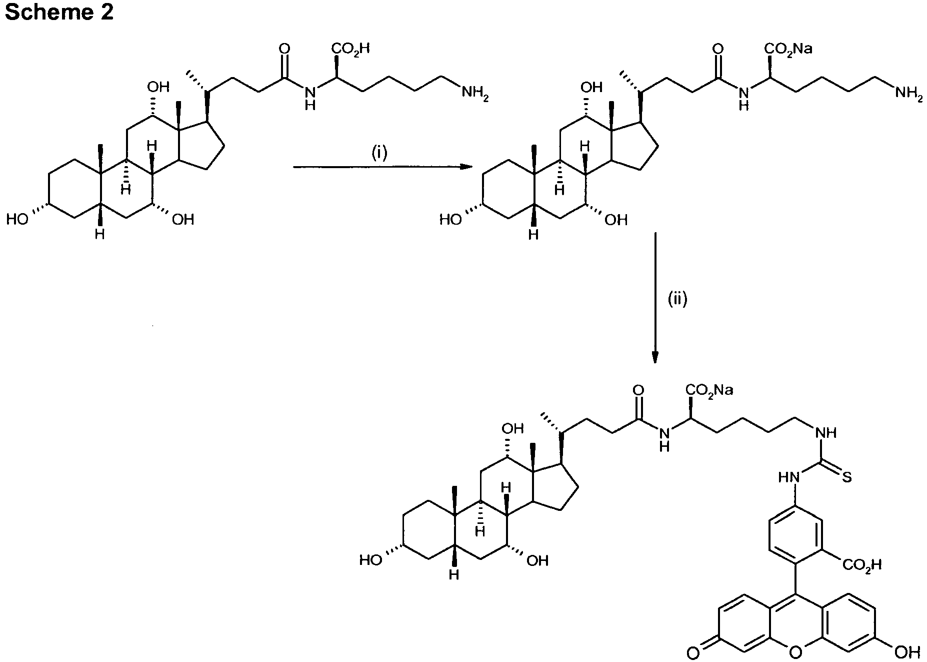

Scheme 2: (i) NaOH, water, methanol; (ii) FITC, chloroform, diisopropylethylamine

### Example 1

### Preparation of 5-/6-nitrofluorescein

### Reactor 1

A reactor previously made inert with nitrogen was charged with methanesulphonic acid (2.43 L). 4-nitrophthalic acid (600 g) was added, and the temperature maintained between 20 -25 °C. Following agitation for 10 minutes, resorcinol (benzene-1,3-diol) (657 g) was added and the reaction mixture heated cautiously to 70 °C then to 95-100 °C. The reaction mixture was stirred at 95-100 °C and stirring and heating was continued for a minimum of 2 hours. The reaction mixture was sampled every 2 hours for in-process analysis. Following reaction completion, indicated by a limit of not greater than 2% 4-nitrophthalic acid by HPLC, the reaction was cooled to 60 - 70 °C.

### Reactor 2

A second reactor was charged with cold (0-10 °C), deionised water (7.2 L). The reaction mixture at 60-70 °C was charged to the cold water, keeping the temperature of the solution at less than 30 °C. The mixture was agitated for a minimum of 30 minutes and the precipitated product isolated by filtration. The isolated material was washed with deionised water (0.36 L) and pulled dry under vacuum.

The damp product was recharged to the reactor, deionised water (4.8 L) added and the mixture heated to 95-100 °C with agitation for 30 minutes. The suspension was cooled to 20-30 °C and the solid product isolated by filtration, washed with deionised water (0.48 L) and pulled dry. The damp product was analysed by proton NMR to determine the methanesulphonic acid ester content. A methanesulphonic acid ester content of less than 5 % relative to the product is preferred. The hydrolysis procedure was repeated until the ester content was within this limit. The solid was transferred to a vacuum oven and dried at 65-70 °C until constant weight to give the product as a dark orange solid.
m.p = 346-349 °C
¹H (DMSO-D₆) 6.54-6.58 (6H, m, 6-isomer) 6.58-6.72 (6H, m, 5-isomer), 7.57 (1H, d, 5-isomer), 8.1 (1H, d, 6-isomer), 8.26 (1H, d, 6-isomer), 8.5 (1H, dd, 6-isomer), 8.57 (1H, dd, 5-isomer), 8.66 (1H, d, 5-isomer), (1H, m), 4.15 (1H, d)
m/z = 378.1 (M+1)

In the above example, the precipitated product was isolated from the reaction mixture by filtration. It will be understood that isolation of the precipitated product may also be achieved by centrifugation.

## Claims

1. A process for the preparation of a mixture of 3',6'-dihydroxy-6-nitrospiro[2-benzofuran-3,9'-xanthene]-1-one and 3',6'-dihydroxy-5-nitrospiro[2-benzofuran-3,9'-xanthene]-1-one comprising the steps of:-
(a) reacting 4-nitrophthalic acid or 4-nitrophthalic anhydride with benzene-1,3-diol in methanesulphonic acid;
(b) quenching the reaction in step (a) with a solvent to precipitate product;
(c) isolating the precipitate;
(d) heating the precipitate in water in order to hydrolyse any methansulphonic acid ester present.

2. A process as claimed in claim 1 wherein the reaction in step (a) is carried out at 60° to 120°C.

3. A process as claimed in claim 2 wherein the reaction in step (a) is carried out at above 90°C.

4. A process as claimed in any preceding claim wherein the reaction in step (a) is carried out at 95°C to 100°C.

5. A process as claimed in any of claims 1 to 4 inclusive wherein the methanesulphonic acid is not less than 95% w/w.

6. A process as claimed in claim 5 wherein the methanesulphonic acid is not less than 98% w/w.

7. A process as claimed in any preceding claim wherein the reaction in step (a) is quenched by adding the reaction to the solvent.

8. A process according to claim 7 wherein the reaction in step (a) is quenched with water to precipitate the product.

9. A process according to claim 8 wherein the ratio of water to methanesulphonic acid is 4.5 w/w or less.

10. A process according to claim 9 wherein the ratio of water to methanesulphonic acid is 3 w/w or less.

11. A process as claimed in claim 9 or claim 10 wherein the temperature of the water before the reaction is quenched is 0° to 10°C.

12. A process as claimed in any of claims 9 to 11 inclusive wherein the temperature of the water is maintained at 30°C or less during the period in which the reaction is quenched.

13. A process as claimed in any preceding claim wherein the precipitated product in step (c) is isolated by filtration.

14. A process as claimed in any of claims 1 to 12 inclusive wherein the precipitated product in step (c) is isolated by centrifugation.

15. A process as claimed in any preceding claim wherein the precipitate in step (d) is heated in water at 80° to 100°C.

16. A process as claimed in any preceding claim wherein the precipitate in step (d) is heated with agitation.

17. A process according to any preceding claim wherein the precipitate from step (d) is isolated and wherein the step of heating the precipitate in water is repeated.

18. A process according to Claim 17 wherein the step of heating the precipitate in water is repeated until the level of methanesulponic acid ester present is 5% or less.

19. A process as claimed in any preceding claim wherein the product from step (d) is subjected to drying in a vacuum.

20. A process as claimed in claim 19 wherein the produce is dried at between room temperature and 70°C.

21. A process as claimed in any preceding claim wherein the ratio of methanesulphonic acid to 4-nitrophthalic acid/4-nitrophthalic anhydride in step (a) is 2 to 10 v/w.

22. A process as claimed in claim 21 wherein the ratio of methanesulphonic acid to 4-nitrophthalic acid/4-nitrophthalic anhydride is 3 to 5 v/w.

## Patentansprüche

1. Verfahren zur Zubereitung einer Mischung von 3',6'-Dihydroxy-6-Nitrospiro[2-Benzofuran-3,9'-Xanthen]-1-eins und 3',6'-Dihydroxy-5-Nitrospiro[2-Benzofuran-3,9'-Xanthen]-1-eins mit den folgenden Schritten:
(a) Reagieren von 4-Nitrophtalsäure oder 4-Nitrophtalanhydrid mit Benzen-1,3-Diol in Methansulfonsäure;
(b) Quenchen der Reaktion im Schritt (a) mit einem Lösungsmittel zum Ausfällen von Produkt;
(c) Isolieren des ausgefällten Produkts;
(d) Erhitzen des ausgefällten Produkts in Wasser zur Hydrolyse von irgendwelchem vorliegenden Methansulfonsäureester.

2. Verfahren nach Anspruch 1, bei dem die Reaktion im Schritt (a) bei 60° bis 120 °C ausgeführt wird.

3. Verfahren nach Anspruch 2, bei dem die Reaktion im Schritt (a) bei mehr als 90 °C ausgeführt wird.

4. Verfahren nach einem der vorhergehenden Ansprüche, bei dem die Reaktion im Schritt (a) bei 95° bis 100 °C ausgeführt wird.

5. Verfahren nach einem der Ansprüche 1 bis 4, bei dem die Methansulfonsäure nicht weniger als 35% w/w ist.

6. Verfahren nach Anspruch 5, bei dem die Methansulfonsäure nicht weniger als 98% w/w ist.

7. Verfahren nach einem der vorhergehenden Ansprüche, bei dem die Reaktion im Schritt (a) durch Hinzufügen der Reaktion zu dem Lösungsmittel gequencht wird.

8. Verfahren nach Anspruch 7, bei dem die Reaktion im Schritt (a) mit Wasser gequencht wird, um das Produkt auszufällen.

9. Verfahren nach Anspruch 8, bei dem das Verhältnis von Wasser zu Methansulfonsäure 4,5 w/w oder weniger ist.

10. Verfahren nach Anspruch 9, bei dem das Verhältnis von Wasser zu Methansulfonsäure 3 w/w oder weniger ist.

11. Verfahren nach Anspruch 9 oder Anspruch 10, bei dem die Temperatur des Wassers vor dem Quenchen der Reaktion 0 bis 10 °C ist.

12. Verfahren nach einem der Ansprüche 9 bis 10 einschließlich, bei dem die Temperatur des Wassers auf 50 °C oder weniger während der Periode gehalten wird, in der die Reaktion gequencht wird.

13. Verfahren nach einem der vorhergehenden Ansprüche, bei dem das ausgefällte Produkt im Schritt (c) durch Filtration isoliert wird.

14. Verfahren nach einem der Ansprüche 1 bis 12 einschließlich, bei dem das ausgefällte Produkt im Schritt (c) durch Zentrifugation isoliert wird.

15. Verfahren nach einem der vorhergehenden Ansprüche, bei dem das ausgefällte Produkt im Schritt (d) in Wasser auf 80 ° bis 100 °C erhitzt wird.

16. Verfahren nach einem der vorhergehenden Ansprüche, bei dem das ausgefällte Produkt im Schritt (d) unter Rühren erhitzt wird.

17. Verfahren nach einem der vorhergehenden Ansprüche, bei dem das ausgefällte Produkt aus dem Schritt (d) isoliert wird, und bei dem der Schritt des Erhitzens des ausgefällten Produktes in Wasser wiederholt wird.

18. Verfahren nach Anspruch 17, bei dem der Schritt des Erhitzens des ausgefällten Produkts in Wasser wiederholt wird, bis der Pegel des vorliegenden Methansulfonsäureesters 5% oder weniger ist.

19. Verfahren nach einem der vorhergehenden Ansprüche, bei dem das Produkt aus dem Schritt (d) einer Trocknung in einem Vakuum unterworfen wird.

20. Verfahren nach Anspruch 19, bei dem das Produkt bei zwischen Raumtemperatur und 70 °C getrocknet wird.

21. Verfahren nach einem der vorhergehenden Ansprüche, bei dem das Verhältnis der Methansulfonsäure zu 4-Nitrophtalsäure/4-Nitrophtalanhydrid im Schritt (a) 2 bis 10 v/w ist.

22. Verfahren nach Anspruch 21, bei dem das Verhältnis der Methansulfonsäure zum 4-Nitrophtalsäure/4-Nitrophtalanhydrid 3 bis 5 v/w ist.

## Revendications

1. Procédé de préparation d'un mélange de 3',6'-dihydroxy-6-nitrospiro[2-benzofuran-3,9'-xanthène]-1-one et de 3',6'-dihydroxy-5-nitrospiro[2-benzofuran-3,9'-xanthène]-1-one comprenant les étapes suivantes :
(a) faire réagir un acide 4-nitrophtalique ou un anhydride 4-nitrophtalique avec un benzène-1,3-diol dans de l'acide méthanesulfonique ;
(b) neutraliser la réaction de l'étape (a) avec un solvant pour précipiter le produit ;
(c) isoler le précipité ;
(d) chauffer le précipité dans l'eau pour hydrolyser tout ester d'acide méthanesulfonique présent.

2. Procédé tel que revendiqué dans la revendication 1 dans lequel la réaction dans l'étape (a) est mise en oeuvre à une température de 60 à 120 °C.

3. Procédé tel que revendiqué dans la revendication 2 dans lequel la réaction dans l'étape (a) est mise en oeuvre à une température supérieure à 90 °C.

4. Procédé tel que revendiqué dans l'une quelconque des revendications précédentes dans lequel la réaction dans l'étape (a) est mise en oeuvre à une température de 95 à 100 °C.

5. Procédé tel que revendiqué dans l'une quelconque des revendications 1 à 4 incluse dans lequel l'acide méthanesulfonique n'est pas inférieur à 95 % p/p.

6. Procédé tel que revendiqué dans la revendication 5 dans lequel l'acide méthanesulfonique n'est pas inférieur à 98 % p/p.

7. Procédé tel que revendiqué dans l'une quelconque des revendications précédentes dans lequel la réaction dans l'étape (a) est neutralisée par ajout de la réaction dans le solvant.

8. Procédé selon la revendication 7 dans lequel la réaction dans l'étape (a) est neutralisée avec de l'eau pour précipiter le produit.

9. Procédé selon la revendication 8 dans lequel le rapport de l'eau à l'acide méthanesulfonique est de 4,5 p/p ou moins.

10. Procédé selon la revendication 9 dans lequel le rapport de l'eau à l'acide méthanesulfonique est de 3 p/p ou moins.

11. Procédé tel que revendiqué dans la revendication 9 ou la revendication 10, dans lequel la température de l'eau avant la neutralisation de la réaction est de 0 à 10 °C.

12. Procédé tel que revendiqué dans l'une quelconque des revendications 9 à 11 incluse, dans lequel la température de l'eau est maintenue à 30 °C ou moins pendant la période où la réaction est neutralisée.

13. Procédé tel que revendiqué dans l'une quelconque des revendications précédentes, dans lequel le produit précipité dans l'étape (c) est isolé par filtration.

14. Procédé tel que revendiqué dans l'une quelconque des revendications 1 à 12 incluse dans lequel le produit précipité dans l'étape (c) est isolé par centrifugation.

15. Procédé tel que revendiqué dans l'une quelconque des revendications incluse dans lequel le précipité dans l'étape (d) est chauffé dans l'eau de 80 à 100 °C.

16. Procédé tel que revendiqué dans l'une quelconque des revendications précédentes, dans lequel le précipité dans l'étape (d) est chauffé sous agitation.

17. Procédé selon l'une quelconque des revendications précédentes, dans lequel le précipité obtenu à l'étape (d) est isolé et dans lequel l'étape de chauffage du précipité dans l'eau est répétée.

18. Procédé selon la Revendication 17 dans lequel l'étape de chauffage du précipité dans l'eau est répétée jusqu'à ce que le niveau d'ester d'acide méthanesulfonique présent soit de 5 % ou moins.

19. Procédé tel que revendiqué dans l'une quelconque des revendications précédentes, dans lequel le produit de l'étape (d) est soumis à un séchage sous vide.

20. Procédé tel que revendiqué dans la revendication 19, dans lequel le produit est séché à une température entre la température ambiante et 70 °C.

21. Procédé tel que revendiqué dans l'une quelconque des revendications précédentes, dans lequel le rapport de l'acide méthanesulfonique à l'acide 4-nitrophtalique/anhydride 4-nitrophtalique dans l'étape (a) est de 2 à 10 v/p.

22. Procédé tel que revendiqué dans la revendication 21, dans lequel le rapport de l'acide méthanesulfonique à l'acide 4-nitrophtalique/anhydride 4-nitrophtalique est de 3 à 5 v/p.
